# EUROPÄISCHE PATENTSCHRIFT

(11) **EP 0 820 436 B1**
(45) Veröffentlichungstag und Bekanntmachung des Hinweises auf die Patenterteilung: **12.12.2001**
(21) Anmeldenummer: 96910928.9
(22) Anmeldetag: 25.03.1996
(51) Int. Cl.: C07C 251/48, C07C 69/734, C07D 239/52, A01N 37/50, A01N 37/36, A01N 43/54, C07C 235/78, C07C 69/738, C07C 43/00, C07F 9/40, C07D 239/34

(54) **SUBSTITUIERTE CYCLOALKENE UND IHRE VERWENDUNG ALS MIKROBIZIDE, INSBESONDERE ALS FUNGIZIDE**
SUBSTITUTED CYCLOALKENES AND THEIR USE AS MICROBICIDES, ESPECIALLY AS FUNGICIDES
CYCLOALCENES SUBSTITUES ET LEUR UTILISATION COMME MICROBICIDES, NOTAMMENT COMME FONGICIDES

(30) Priorität: 06.04.1995 DE 19512886
(43) Veröffentlichungstag der Anmeldung: 28.01.1998
(73) Patentinhaber: BAYER AG, 51368 Leverkusen (DE)
(72) Erfinder: GAYER, Herbert, D-40789 Monheim (DE); GERDES, Peter, D-52080 Aachen (DE); TIEMANN, Ralf, D-51375 Leverkusen (DE); DUTZMANN, Stefan, D-40721 Hilden (DE); STENZEL, Klaus, D-40595 Düsseldorf (DE)
(86) Internationale Anmeldenummer: EP9601309
(87) Internationale Veröffentlichungsnummer: WO9631463

(56) Entgegenhaltungen:
- EP-A- 0 421 102
- EP-A- 0 468 684
- EP-A- 0 477 631
- EP-A- 0 582 925
- EP-A- 0 617 011
- DE-A- 2 705 881
- DE-A- 4 012 792
- US-E- R E33 989
- TETRAHEDRON LETT. (1991), 32(24), 2779-82, XP002010398 KANG, HAN YOUNG ET AL: "Intramolecular [3+2] nitrone-alkyne cycloaddition"
- CHEMICAL ABSTRACTS, vol. 65, no. 13, 19.Dezember 1966 Columbus, Ohio, US; abstract no. 19999h, J. LUBOCHINSKY ET AL.: "Preparation of conjugated .alpha.-oxoesters" XP002010401 & COMPT. REND., SER. C, Bd. 263, Nr. 11, 1966, Seiten 732-4,
- HELVETICA CHIMICA ACTA, Bd. 71, 1988, BASEL CH, Seiten 168-194, XP002010400 S. E. DENMARK ET AL.: "19. Silicon directed Nazarov cyclizations"
- JOURNAL OF THE CHEMICAL SOCIETY, PERKIN TRANSACTIONS 1, Nr. 3, 1.März 1986, Seiten 515-520, XP000567075 BROOKE G M ET AL: "PARTIALLY FLUORINATED HETEROCYCLIC COMPOUNDS. PART 22.1 THE PREPARATION OF ALLYL 2,5,6-TRIFLUOROPYRIMIDIN-4-YL ETHER AND RELATED COMPOUNDS AND A STUDY OF THEIR CLAISEN REARRANGEMENT REACTIONS. A NEW ROUTE TO 5-FLUOROURACIL AND BARBITURIC ACID DERIVATIVES"
- J. AM. CHEM. SOC. (1995), 117(43), 10635-44, XP002010399 NAKATANI, KAZUHIKO ET AL: ".alpha.-Diazo Ketones as Photochemical DNA Cleavers: A Mimic for the Radical Generating System of Neocarzinostatin Chromophore"

## Beschreibung

Die Erfindung betrifft neue substituierte Cycloalkene, mehrere Verfahren zu ihrer Herstellung, sowie neue Zwischenprodukte und mehrere Verfahren zu deren Herstellung.

Es ist bekannt, daß bestimmte substituierte Cycloalkene fungizide Eigenschaften aufweisen [vgl. z. B. EP-A 421102].

Die Wirkung dieser Verbindungen ist jedoch in vielen Fällen unbefriedigend.

Es wurden nun die neuen substituierten Cycloalkene der allgemeinen Formel (I) gefunden, 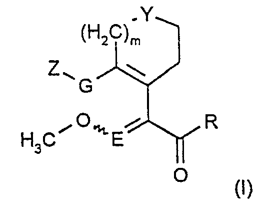 in welcher
- E: für Stickstoff steht,
- G: für -T-Ar¹-Q- steht, wobei
- Q: für Sauerstoff steht,
- Ar¹: für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
- T: für eine Einfachbindung, für Sauerstoff, Schwefel, -CH₂-O-, CH₂-S-, Methylen, Ethylen oder Propylen steht und
- m: für 0 oder 1 steht,
- R: für Methoxy, steht,
- Y: für Methylen steht und
- Z: für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy öder jeweils gegebenenfalls einfach bis vierfach, gleich oder verschieden durch Fluor, Chlor, Methyl, Trifluormethyl oder Ethyl, bevorzugt Fluor, substituiertes, jeweils zweifach verknüpftes Methylendioxy oder Ethylendioxy, substituiertes Phenyl, Pyridyl oder Thienyl steht.
Weiterhin wurde gefunden, daß man die neuen substituierten Cycloalkene der allgemeinen Formel (I) erhält, wenn man
a) Ketoverbindungen der allgemeinen Formel (IX), 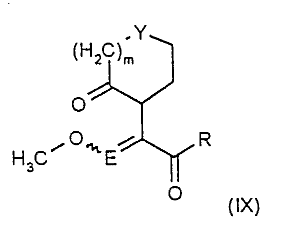 in welcher
   - E, m, Y und R: die oben angegebene Bedeutung haben,
   mit Halogenverbindungen der allgemeinen Formel (X),

   z―A¹―x² (X)

   in welcher
   - A¹: für eine Einfachbindung, -T-Ar¹- oder -CH₂- steht und
   - T, Ar¹ und Z: die oben angegebene Bedeutung haben und
   - X²: für Halogen oder Alkysulfonyl steht,
   gegebenenfalls in Gegenwart eines Säureakzeptors und gegebenenfalls in Gegenwart eines Verdünnungsmittels, umsetzt, oder wenn man
b) Halogenverbindungen der allgemeinen Formel (XI), 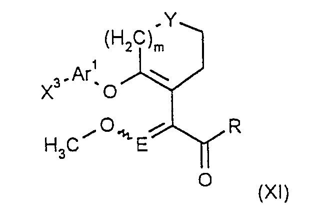 in welcher
   - m, Ar¹, E, R und Y: die oben angegebene Bedeutung haben und
   - X³: für Halogen oder Alkylsulfonyl steht,
   mit Hydroxy- bzw. Mercaptoverbindungen der allgemeinen Formel (IV),

   Z-Q¹-H (IV)

   in welcher
   - Q¹ und Z: die oben angegebene Bedeutung haben,
   gegebenenfalls in Gegenwart eines Säureakzeptors und gegebenenfalls in Gegenwart eines Verdünnungsmittels, umsetzt.

Schließlich wurde gefunden, daß die neuen substituierten Cycloalkene der allgemeinen Formel (I) sehr starke fungizide Wirkung zeigen.

Die erfindungsgemäßen Verbindungen können, ebenso wie die erfindungsgemäßen Zwischenprodukte, gegebenenfalls als Mischungen verschiedener möglicher isomerer Formen, insbesondere von Stereoisomeren, wie z. B. E- und Z-Isomeren, gegebenenfalls aber auch von Tautomeren vorliegen. Es werden sowohl die E- als auch die Z-Isomeren, beliebige Mischungen dieser Isomeren, sowie die möglichen tautomeren Formen beansprucht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Beispiele für die erfindungsgemäßen Verbindungen sind in der Tabelle 1 aufgeführt:

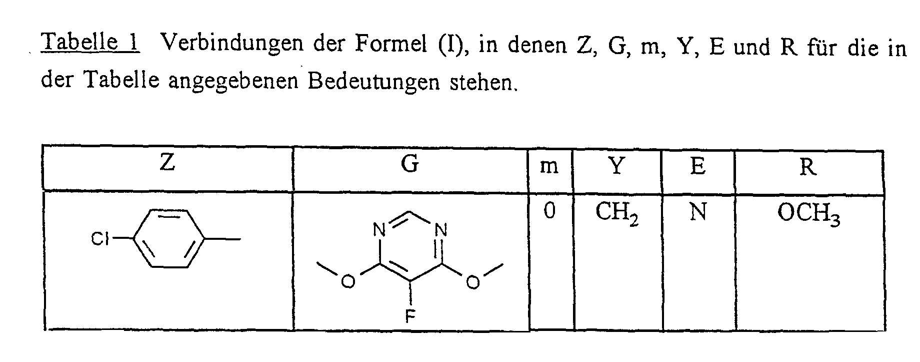

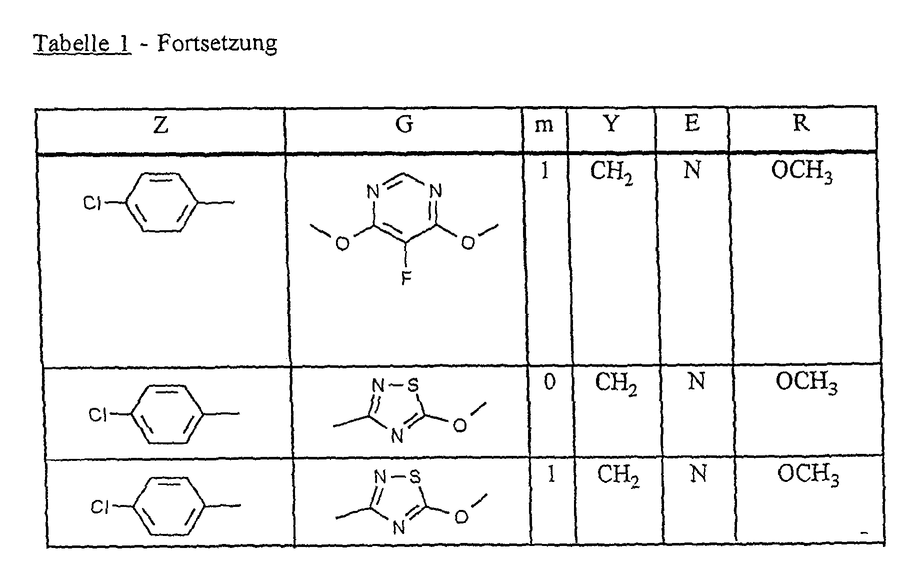

Die zur Durchführung des erfindungsgemäßen Verfahrens a) zur Herstellung der erfindungsgemäßen Verbindungen der Formel (I) als Ausgangsstoffe benötigten Ketoverbindungen sind durch die Formel (IX) allgemein definiert. In dieser Formel (IX) haben m, E, Y und R vorzugsweise bzw. insbesondere diejenige Bedeutung, die bereits im Zusammenhang mit der Beschreibung der erfindungsgemäßen Verbindungen der Formel (I) für m, E, Y und R angegeben wurde.

Die Ketoverbindungen der Formel (IX) werden erhalten (Verfahren a-1a)), wenn man Triketoverbindungen der Formel (XXIV), 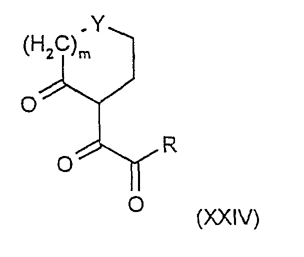 in welcher
- m, R und Y: die oben angegebene Bedeutung haben,
mit Methoxyamin oder dessen Säureaddukten, gegebenenfalls in Gegenwart eines Verdünnungsmittels, wie beispielsweise Methanol, und gegebenenfalls in Gegenwart eines Reaktionshilfsmittels, wie beispielsweise Natriumacetat, bei Temperaturen von 0 bis 150°C, bevorzugt 20 bis 120°C, umsetzt.

Die zur Durchführung des erfindungsgemäßen Verfahrens a-1a) zur Herstellung der Ketoverbindungen der Formel (IX) als Ausgangsstoffe benötigten Triketoverbindungen sind durch die Formel (XXIV) allgemein definiert. In dieser Formel (XXIV) haben m, R und Y vorzugsweise bzw. insbesondere diejenige Bedeutung, die bereits im Zusammenhang mit der Beschreibung der erfindungsgemäßen Verbindungen der Formel (I) für m, R und Y angegeben wurde.

Die Triketoverbindungen der Formel (XXIV) sind bekannt und/oder können nach an sich bekannten Verfahren hergestellt werden (vgl. z. B. J. Org. Chem. (1977), 42(7), 1180-5).

Die weiterhin zur Durchführung des erfindungsgemäßen Verfahrens a) zur Herstellung der erfindungsgemäßen Verbindungen der Formel (I) als Ausgangsstoffe benötigten Halogenverbindungen sind durch die Formel (X) allgemein definiert. In dieser Formel (X) steht A¹ für -T-Ar¹- oder -CH₂-. Ar¹, T und Z haben vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Ar¹, T und Z angegeben wurde. X² steht für Halogen, bevorzugt Fluor, Chlor, Brom oder Iod, oder für Alkylsulfonyl, bevorzugt Methylsulfonyl.

Die Halogenverbindungen der Formel (X) sind größtenteils bekannte Synthesechemikalien und/oder können nach an sich bekannten Verfahren hergestellt werden (vgl. z. B. Khim.-Farm. Zh. (1989), 23(6), 705-7).

Neu und auch Gegenstand der Erfindung sind Halogenverbindungen der allgemeinen Formel (Xa), 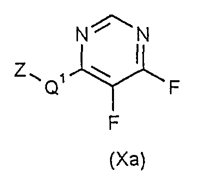 in welcher
- Q¹: für Sauerstoff oder Schwefel steht und
- Z: die oben angegebene Bedeutung hat.

Die Halogenverbindungen der allgemeinen Formel (Xa) werden erhalten, (Verfahren a-1b)), wenn man die bereits weiter oben beschriebenen Hydroxy- bzw. Mercaptoverbindungen der allgemeinen Formel (IV) mit 4,5,6-Trifluorpyrimidin (DE-A 4137291), gegebenenfalls in Gegenwart eines Verdünnungsmittels, wie beispielsweise Methanol, Acetonitril oder Tetrahydrofuran, und gegebenenfalls in Gegenwart eines Säurebindemittels, wie beispielsweise Natriummethylat oder Kalium-t-butylat, bei Temperaturen von 0 bis 150°C, vorzugsweise 0 bis 120°C, umsetzt.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methylisobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sufone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen zwischen -20°C und +150°C, vorzugsweise bei Temperaturen zwischen 0°C und 100°C.

Zur Durchführung des erfindungsgemäßen Verfahrens a) zur Herstellung der Verbindungen der Formel (I) setzt man pro Mol der Ketoverbindung der Formel (IX) im allgemeinen 1 bis 15 Mol, vorzugsweise 1 bis 8 Mol an Halogenverbindung der Formel (X) ein.

Die zur Durchführung des erfindungsgemäßen Verfahrens b) zur Herstellung der erfindungsgemäßen Verbindungen der Formel (I) als Ausgangsstoffe benötigten Halogenverbindungen sind durch die Formel (XI) allgemein definiert. In dieser Formel (XI) haben m, Ar¹, E und Y vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für m, Ar¹, E und Y angegeben wurde. X³ steht für Halogen, bevorzugt Chlor oder Fluor, oder für Alkylsulfonyl, bevorzugt Methylsulfonyl.

Die Halogenverbindungen der Formel (XI) sind erfindungsgemäße Verbindungen und können gemäß dem erfindungsgemäßen Verfahren a) hergestellt werden.

Als Verdünnungsmittel zur Durchführung des erfindungsgemäßen Verfahrens b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methylisobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sufone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren b) wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens b) in einem größeren Bereich variiert werden. Im allgemeinen arbeitet man bei Temperaturen zwischen -20°C und +150°C, vorzugsweise bei Temperaturen zwischen 0°C und 100°C.

Zur Durchführung des erfindungsgemäßen Verfahrens b) zur Herstellung der Verbindungen der Formel (I) setzt man pro Mol der Ketoverbindung der Formel (IX) im allgemeinen 1 bis 15 Mol, vorzugsweise 1 bis 8 Mol an Halogenverbindung der Formel (X) ein.

Die erfindungsgemäßen Verfahren a) und b) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans; Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform:
Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Getreidekrankheiten, wie beispielsweise gegen Erysiphe- und Pseudocercosporella Arten, oder von Krankheiten im Wein-, Obst- und Gemüseanbau, wie beispielsweise gegen Plasmopara-Arten eingesetzt.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

### Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro- N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb,
Cymoxanil, Cyproconazole, Cyprofuram,
Dichlorophen, Diclobutrazol, Diclofluanid, Didomezin, Dicloran, Diethofencarb,
Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanatmethyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A,
Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb,
Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Primiphos A, Profenofos,
Profenophos, Promecarb, Propaphos, Propoxur, Prothiofos, Prothiophos, Prothoat,
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.
Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Beispiel (1)

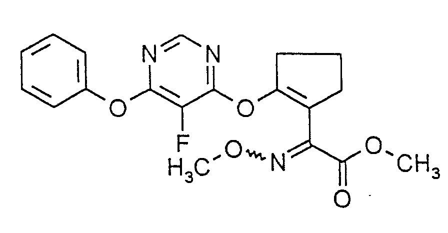

### Verfahren d)

2 g (0,007 Mol) 70%iges 1-Methoximino-1-(2-oxocyclopentan-1-yl)-essigsäuremethylester werden in 10 ml Dimethylformamid mit 0,54 g (0,01 Mol) Natriummethylat (M = 54,02) 15 Minuten bei Raumtemperatur gerührt. Man gibt 2,08 g (0,01 Mol) 5,6-Difluor-4-phenoxypyrimidin zu und rührt 3 Stunden bei Raumtemperatur. Man gießt auf Wasser, extrahiert mit Diethylether, trocknet die organische Phase über Natriumsulfat und engt im Vakuum ein. Der Rückstand wird aus Methanol umkristallisiert. Man erhält 1,43 g (52,5 % der Theorie) 1-Methoximino-1-[2-(5-fluor-6-phenoxy-pyrimid-4-yl)-penten-1-yl]-essigsäuremethylester vom Schmelzpunkt 133 - 135°C

### Herstellung der Ausgangsverbindung,

### Beispiel (IX-1)

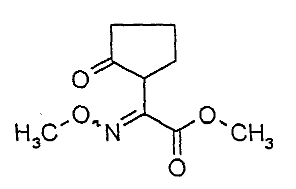

### Verfahren a-1)

3 g (17,6 mMol) 2-Methoxalyl-cyclopentanon (M = 170,17) werden mit 1,5 g (17,95 mMol) Methoxyamin hydrochlorid und 2,5 g (18,1 mMol) Kaliumcarbonat in 17 ml Methanol 1 Stunde unter Rückfluß erhitzt. Man destilliert das Lösungsmittel im Vakuum ab, versetzt mit Wasser und extrahiert mit Dichlormethan. Die organische Phase wird über Natriumsulfat getrocknet und im Vakuum eingeengt. Das Rohprodukt wird im Vakuum destilliert. Man erhält 2,84 g (80,8 % der Theorie) 1-Methoximino-1-(2-oxocyclopentan-1-yl)-essigsäuremethylester vom Siedepunkt 80°C bei 0,4 mb.
¹H-NMR(CDCl₃, TMS): δ = 1,5 - 2,5 (7 H); 3,85 (3 H); 4,06 (3 H) ppm

### Herstellung der Ausgangsverbindung,

### Beispiel (Xa-1)

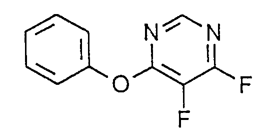

### Verfahren a-1b

Zu einer Lösung von 42,4 g (0,45 Mol) Phenol in 400 ml THF gibt man unter Rühren 50,4 g (0,45 Mol) Kalium-tert.-butylat (M =112,22). Die so bereitete Phenol-Kaliumsalzlösung tropft man zu einer auf 0°C gekühlten Lösung von 80 g (0,6 Mol) 4,5,6-Trifluorpyrimidin in 1 1 THF und läßt 30 Minuten nachrühren. Die Lösung wird im Vakuum eingeengt, der Rückstand mit Wasser versetzt und mit Essigsäureethylester extrahiert. Die organische Phase wird abgetrennt, über Natriumsulfat getrocknet und im Vakuum eingeengt. Das Rohprodukt wird mit Petrolether verrührt und abfiltriert. Man erhält 63,8 g (68,1 % der Theorie) 5,6-Difluor-4-phenoxypyrimidin vom Schmelzpunkt 65 - 66°C.

## Patentansprüche

1. Verbindungen der Formel (I) 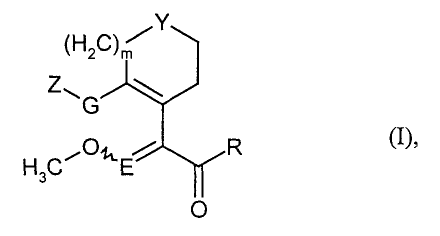 in welcher
E für Stickstoff steht,
G für -T-Ar¹-Q- steht, wobei
Q für Sauerstoff steht,
Ar¹ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht und
m für 0 oder 1 steht,
R für Methoxy steht,
Y für Methylen steht und
Z für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy oder jeweils gegebenenfalls einfach bis vierfach, gleich oder verschieden durch Fluor, Chlor, Methyl, Trifluormethyl oder Ethyl, bevorzugt Fluor, substituiertes, jeweils zweifach verknüpftes Methylendioxy oder Ethylendioxy, substituiertes Phenyl, Pyridyl oder Thienyl steht.

2. Schädlingsbekämpfungsmittel, **gekennzeichnet durch** einen Gehalt an mindestens einer Verbindung der Formel (I) nach Anspruch 1.

3. Verfahren zur Bekämpfung von Schädlingen, **dadurch gekennzeichnet, daß** man Verbindungen der Formel (I) nach Anspruch 1 auf Schädlinge und/oder ihren Lebensraum einwirken läßt.

4. Verwendung von Verbindungen der Formel (I) nach Anspruch 1 zur Bekämpfung von Schädlingen.

5. Verfahren zur Herstellung von Schädlingsbekämpfungsmitteln, **dadurch gekennzeichnet, daß** man Verbindungen der Formel (I) nach Anspruch 1 mit Streckmitteln und/oder oberflächenaktiven Mitteln vermischt.

6. Verfahren zur Herstellung von Verbindungen der Formel (I), **dadurch gekennzeichnet, daß** man
d) Ketoverbindungen der allgemeinen Formel (IX), 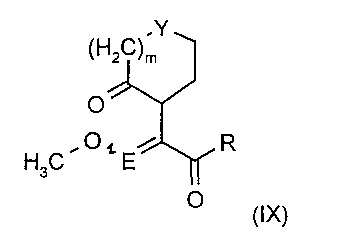 in welcher
E, m, Y und R die in Anspruch 1 angegebene Bedeutung haben,
mit Halogenverbindungen der allgemeinen Formel (X),
Z―A¹-X² (X)
in welcher
A¹ für eine Einfachbindung, -T-Ar¹- oder -CH₂- steht und
T, Ar¹ und Z die in Anspruch 1 angegebene Bedeutung haben und
X² für Halogen oder Alkysulfonyl steht,
gegebenenfalls in Gegenwart eines Säureakzeptors und gegebenenfalls in Gegenwart eines Verdünnungsmittels, umsetzt, oder
e) Halogenverbindungen der allgemeinen Formel (XI), 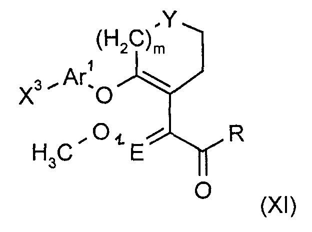 in welcher
m, Ar¹, E, R und Y die in Anspruch 1 angegebene Bedeutung haben und
X³ für Halogen oder Alkylsulfonyl steht,
mit Hydroxy- bzw. Mercaptoverbindungen der allgemeinen Formel (IV),
Z-Q¹-H (IV)
in welcher
Q¹ für Sauerstoff oder Schwefel steht und
Z die in Anspruch 1 angegebene Bedeutung hat,
gegebenenfalls in Gegenwart eines Säureakzeptors und gegebenenfalls in Gegenwart eines Verdünnungsmittels, umsetzt.

7. Verbindungen der Formel (Xa), 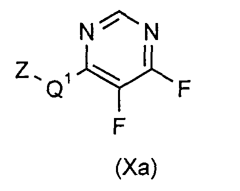 in welcher
Q¹ für Sauerstoff oder Schwefel steht und
Z die in Anspruch 1 angegebene Bedeutung hat.

## Claims

1. Compounds of the formula (I) 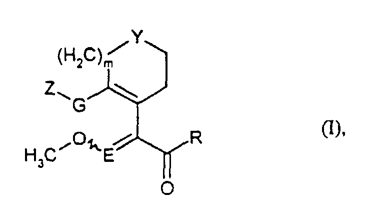 in which
E represents nitrogen,
G represents -T-Ar¹-Q-, where
Q represents oxygen,
Ar¹ represents 1,2,4-thiadiazoldiyl, 1,3,4-thiadiazoldiyl, 1,2,4-oxadiazoldiyl, 1,3,4-oxadiazoldiyl or represents pyridindiyl, pyrimidindiyl or 1,3,5-triazinediyl, each of which is optionally mono- or disubstituted by identical or different substituents from the group consisting of fluorine, chlorine, cyano, methyl, cyclopropyl, methoxy, methylthio, trifluoromethyl, difluoromethoxy, trifluoromethoxy and difluorochloromethoxy,
T represents a single bond, represents oxygen, sulphur, -CH₂-O-, CH₂-S-, methylene, ethylene or propylene and
m represents 0 or 1,
R represents methoxy,
Y represents methylene and
Z represents phenyl, pyridyl or thienyl, each of which is optionally mono- to trisubstituted by identical or different substitutents from the group consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy, difluorochloromethoxy, trifluoroethoxy, trifluoromethoxy and in each case doubly attached methylenedioxy or ethylenedioxy, each of which is optionally mono- to tetrasubstituted by identical or different substituents from the group consisting of fluorine, chlorine, methyl, trifluoromethyl and ethyl, preferably fluorine.

2. Pesticides, **characterized in that** they comprise at least one compound of the formula (I) according to Claim 1.

3. Method for controlling pests, **characterized in that** compounds of the formula (I) according to Claim 1 are allowed to act on pests and/or their habitat.

4. Use of compounds of the formula (I) according to Claim 1 for controlling pests.

5. Process for preparing pesticides, **characterized in that** compounds of the formula (I) according to Claim 1 are mixed with extenders and/or surfactants.

6. Process for preparing compounds of the formula (I), **characterized in that**
a) keto compounds of the general formula (IX) 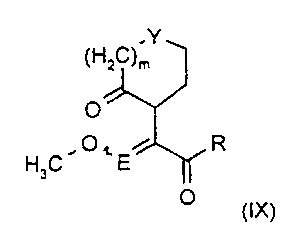 in which
E, m, Y and R are each as defined in Claim 1
are reacted with halogen compounds of the general formula (X)
Z―A¹-X² (X)
in which
A¹ represents a single bond, -T-Ar¹ or -CH₂- and
T, Ar¹ and Z are as defined in Claim 1 and
X² represents halogen or alkylsulphonyl,
if appropriate in the presence of an acid acceptor and if appropriate in the presence of a diluent, or
b) halogen compounds of the general formula (XI), 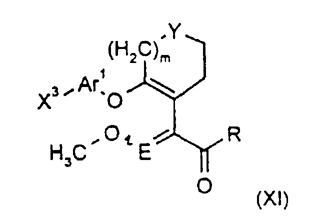 in which
m, Ar¹, E, R and Y are as defined in Claim 1 and
X³ represents halogen or alkylsulphonyl
are reacted with hydroxyl or mercapto compounds of the general formula (IV)
Z-Q¹-H (IV)
in which
Q¹ represents oxygen or sulphur and
Z is as defined in Claim 1,
if appropriate in the presence of an acid acceptor and if appropriate in the presence of a diluent.

7. Compounds of the formula (Xa) 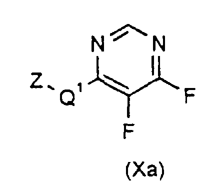 in which
Q¹ represents oxygen or sulphur and
Z is as defined in Claim 1.

## Revendications

1. Composés de formule (I) 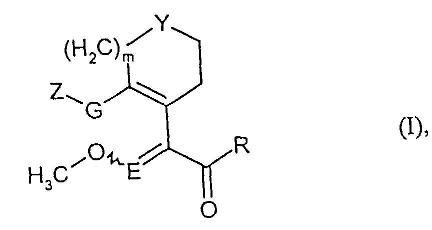 dans laquelle
E représente l'azote,
G représente un groupe -T-Ar¹-Q- dans lequel
Q est l'oxygène,
Ar¹ représente un groupe 1,2,4-thiadiazolediyle, 1,3,4-thiadiazolediyle, 1,2,4-oxadiazolediyle, 1,3,4-oxadiazolediyle ou un groupe pyridinediyle, pyrimidinediyle ou 1,3,5-triazinediyle portant chacun, le cas échéant, un ou deux substituants, identiques ou différents, fluor, chlore, cyano, méthyle, cyclopropyle, méthoxy, méthylthio, trifluorométhyle, difluorométhoxy, trifluorométhoxy, difluorochlorométhoxy,
T est une liaison simple, l'oxygène, le soufre, un groupe -CH₂-O-, CH₂-S-, méthylène, éthylène ou propylène et
m a la valeur 0 ou 1,
R représente un groupe méthoxy,
Y est un groupe méthylène et
Z est un groupe phényle, pyridyle ou thiényle portant chacun, le cas échéant, un à trois substituants, identiques ou différents, fluor, chlore, brome, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, difluorochlorométhoxy, trifluoréthoxy, trifluorométhoxy ou bien méthylènedioxy ou éthylènedioxy ayant chacun deux liaisons et portant chacun, le cas échant, un à quatre substituants, identiques ou différents, fluor, chlore, méthyle, trifluorométhyle ou éthyle, de préférence fluor.

2. Compositions pesticides, **caractérisées par** une teneur en au moins un composé de formule (I) suivant la revendication 1.

3. Procédé pour combattre des parasites, **caractérisé en ce qu'**on fait agir des composés de formule (I) suivant la revendication 1 sur les parasites et/ou sur leur milieu.

4. Utilisation de composés de formule (I) suivant la revendication 1 pour combattre des parasites.

5. Procédé de préparation de compositions pesticides, **caractérisé en ce qu'**on mélange des composés de formule (I) suivant la revendication 1 avec des diluants et/ou des agents tensio-actifs.

6. Procédé de production de composés de formule (I), **caractérisé en ce qu'**
a) on fait réagir des composés cétoniques de formule générale (IX) 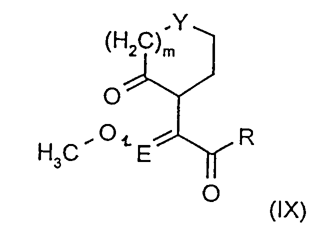 dans laquelle
E, m, Y et R ont la définition indiquée dans la revendication 1,
avec des composés halogénés de formule générale (X)
Z―A¹-X² (X)
dans laquelle
A¹ représente une liaison simple, un groupe -T-Ar¹- ou -CH₂- et
T, Ar¹ et Z ont la définition indiquée dans la revendication 1 et
X² représente un halogène ou un groupe alkylsulfonyle, le cas échéant en présence d'un accepteur d'acide et en présence éventuelle d'un diluant, ou bien
b) on fait réagir des composés halogénés de formule générale (IX) 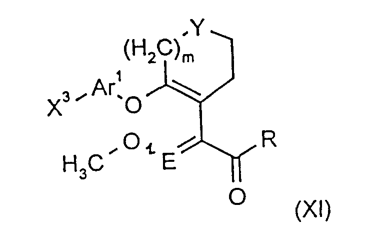 dans laquelle
m, Ar¹, E, R et Y ont la définition indiquée dans la revendication 1 et
X³ représente un halogène ou un groupe alkylsulfonyle, avec des composés hydroxylés ou des composés mercapto de formule générale (IV)
Z-Q¹-H (IV)
dans laquelle
Q¹ est l'oxygène ou le soufre et
Z a la définition indiquée dans la revendication 1,
le cas échéant en présence d'un accepteur d'acide et en présence éventuelle d'un diluant.

7. Composés de formule (Xa) 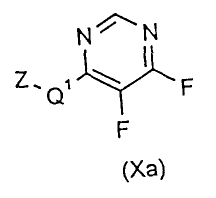 dans laquelle
Q¹ est l'oxygène ou le soufre et
Z a la définition indiquée dans la revendication 1.
